# EUROPEAN PATENT SPECIFICATION

(11) **EP 2 919 862 B1**
(45) Date of publication and mention of the grant of the patent: **14.09.2016**
(21) Application number: 12791671.6
(22) Date of filing: 16.11.2012
(51) Int. Cl.: A61Q 19/10, A61K 8/39, A61K 8/86

(54) **CLEANSING COMPOSITION WITH AN ALKOXYLATED ALKYL ETHER**
REINIGUNGSZUSAMMENSETZUNG MIT EINEM ALKOXYLIERTEN ALKYLETHER
COMPOSITION DÉMAQUILLANTE COMPRENANT UN ÉTHER D'ALKYLE ALCOXYLÉ

(43) Date of publication of application: 23.09.2015
(73) Proprietor: Colgate-Palmolive Company, New York, NY 10022 (US)
(72) Inventor: FAN, Aixing, Bridgewater, New Jersey 08807 (US); KILPATRICK-LIVERMAN, LaTonya, Princeton, New Jersey 08542 (US); MASTERS, James G., Ringoes, New Jersey 08551 (US); SIMPSON, Edward, Monmouth Junction, New Jersey 08852 (US); WU, Joanna, Hillsborough, New Jersey 08844 (US)
(74) Representative: Jenkins, Peter David
(86) International application number: PCT/US2012/065394
(87) International publication number: WO 2014/077826

(56) References cited:
- WO-A1-95/03781
- WO-A1-96/06595
- WO-A1-98/50005
- WO-A1-2004/108105
- WO-A1-2005/046633
- WO-A1-2006/058755
- WO-A1-2008/063501
- WO-A2-2004/108104
- US-A- 4 285 826
- US-A1- 2003 166 497
- US-A1- 2012 070 341
- US-A1- 2012 076 747
- DATABASE GNPD [Online] MINTEL; 1 November 2008 (2008-11-01), "Dove Go Fresh", XP002703610, Database accession no. 998505

## Description

### FIELD OF THE INVENTION

The present invention relates to cleansing compositions with an alkoxylated alkyl ether.

### BACKGROUND OF THE INVENTION

When designing liquid cleansing compositions for consumers, there are several properties of the composition that are adjusted to meet consumer desires and expectations. Consumers may desire a cleansing composition with an increased viscosity. To achieve this viscosity, the composition can be thickened, such as with salt. When viscosity is increased, the composition can become stringy when dispensed. Stringiness is an increased time to break a stream of a composition after the flow has stopped. When dispensed from a container, this can result in a drip from the dispenser. It is desired to reduce stringiness.

Also, consumers desire that cleansing compositions deliver a desired amount of foam. It would be desirable to increase foam volume. Also, emollients may be desired to moisturize skin, but emollients have a tendency to reduce foam because of their oiliness. Also, it would be desirable to deliver more fragrance intensity from a composition so that less fragrance can be used but still deliver the same intensity to save costs. In addition, a micellar system that allows enhanced solubilization of oils and fragrances could be a desirable attribute which allows oil or fragrance incorporation at higher dosages without phase separation.

XP-002703610 discloses a shower cream. WO 95/03781 and WO 96/06595 disclose personal cleansing compositions. US 4285826 discloses toilet soap bars imparting improved moisturizing and skin feel characteristics.

### BRIEF SUMMARY OF THE INVENTION

In one aspect, the present invention provides a liquid cleansing composition according to claim 1. In another aspect, the present invention provides a method according to claim 11. A still further aspect of the invention provides a use according to claim 14. Also described is a liquid cleansing composition comprising a cleansing effective amount of surfactant and an alkoxylated alkyl ether having 5 to 20 alkoxy moieties and a C₃ to C₁₂ alkyl.

Also described is a method of cleansing skin comprising cleansing the skin with the liquid cleansing composition, and optionally rinsing the skin with water.

Also described is a method comprising adding an alkoxylated alkyl ether having 5 to 20 alkoxy moieties and a C₃ to C₁₂ alkyl to an aqueous, liquid cleansing composition for at least one of: reduce stringiness of the cleansing composition, increase fragrance bloom from the cleansing composition, increase oil solubilization, increase foam volume of the cleansing composition, or decrease dry skin.

Also described is the use of an alkoxylated alkyl ether having 5 to 20 alkoxy moieties and a C₃ to C₁₂ alkyl in an aqueous, liquid cleansing composition for at least one of: reduce stringiness of the cleansing composition, increase fragrance bloom from the cleansing composition, increase oil solubilization, increase foam volume of the cleansing composition, or decrease dry skin.

In certain embodiments, the alkoxy can be PEG or PPG. In certain embodiments, the alkoxylated alkyl ether is PPG-14 butyl ether.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION OF THE INVENTION

The following description of the preferred embodiment(s) is merely exemplary in nature and is in no way intended to limit the invention, its application, or uses.

The alkoxylated alkyl ether having 5 to 20 alkoxy moieties and a C₃ to C₁₂ alkyl has a formula of ALKOXY-X C₃ to C₁₂ ether, wherein Alkoxy is PEG (polyethylene glycol) or PPG (polypropylene glycol) and X is a number from 5 to 20. In certain embodiments, the alkoxy is propoxylated. In other embodiments, the alkyl is C₃ to C₆. In other embodiments, the alkyl is butyl. In certain embodiments, the alkoxylated alkyl ether has 12 to 20 alkoxy moieties, optionally 12 to 16 alkoxy moieties. In certain embodiments, the alkoxylated alkyl ether is PPG-14 butyl ether.

In certain embodiments, references to PPG-14 butyl ether refer to the commercially available PPG-14 butyl ether, which is a mixture of PPG-14 butyl ether and PPG-15 butyl ether.

The alkoxylated alkyl ether can be included in liquid cleansing compositions, such as body washes/shower gels, liquid hand soaps, or shampoos. The alkoxylated alkyl ether can reduce stringiness of the composition, increase foam volume, increase fragrance blooming, increase oil solubilization, and moisturize skin. It has been found that alkoxylated alkyl ether can reduce stringiness of surfactant compositions while keeping the viscosity relatively close to the viscosity of the original composition. Foam volume can be increased by the addition of alkoxylated alkyl ether. Because alkoxylated alkyl ether is oily, it would be expected that oil would reduce the volume of foam. It has been found, however, that alkoxylated alkyl ether can increase the volume of foam. Alkoxylated alkyl ether can increase solubilization of oils (such as fragrance oils) in the composition. Generally, when the amount of oil is increased in the composition, the increased oil content causes the composition to become more opaque. The alkoxylated alkyl ether allows for an increase in the amount of oil without an increase of opacity.

The alkoxylated alkyl ether having 5 to 20 alkoxy moieties and a C₃ to C₁₂ alkyl can be included in cleansing compositions in any desired amounts. According to the present invention, the alkoxylated alkyl ether is present in the composition in an amount of 0.1 to 0.5% by weight of the composition. Embodiments of cleansing compositions including alkoxylated alkyl ether in amounts outside this range are provided for reference. In certain embodiments, the amount is 0.01 to 10% by weight of the composition. In other embodiments, the amount is 0.01 up to 9, 8, 7, 6, 5, 4, 3, 2, 1.5, 1, 0.9, 0.8, 0.7, 0.6, or 0.5% by weight of the composition. In other embodiments, the amount is at least 0.025, at least 0.05, at least 0.1, at least 0.2, at least 0.3, at least 0.4, or at least 0.5 up to 9, 8, 7, 6, 5, 4, 3, 2, or 1% by weight. In certain embodiments, the amount is 0.025, 0.05, 0.1, 0.2, 0.3, 0.4, 0.5, 0.6, 0.7, 0.8, 0.9, 1, 1.5, 2, 3, 4, 5, 6, 7, 8, or 9% by weight of the composition. In other embodiments, the amount is 0.05 to 8%, 0.05 to 5%, 0.1 to 5%, 0.1 to 2%, 0.1 to 1%, 0.1 to 0.5%, or 0.1 to 0.3% by weight of the composition. In one embodiment, the amount is 0.3% by weight of the composition. In certain embodiments, the amount is up to 0.5% by weight. The alkoxylated alkyl ether above this amount will tend to require the composition to be adjusted for viscosity or foam generation. Above this level, the viscosity and the level of foam will be decreased. The alkoxylated alkyl ether can be included in amounts up to 9 weight % without causing phase separation, and this can be used for many embodiments, but when targeting higher viscosity or foam generation in other embodiments, the level of alkoxylated alkyl ether can be kept below 0.5 weight %.

It has been discovered that an alkoxylated alkyl ether having 5 to 20 alkoxy moieties and a C₃ to C₁₂ alkyl can be included up to 9% by weight of the composition with the composition remaining clear. This can be useful for moisturizing skin with the composition being a clear composition. Typically, most emollients will opacify a cleansing composition at higher levels.

For achieving increased foam, reducing stringiness, or increasing oil solubilization, the amount of an alkoxylated alkyl ether having 5 to 20 alkoxy moieties and a C₃ to C₁₂ alkyl will be amounts up to about 0.5% by weight. At lower levels, an alkoxylated alkyl ether having 5 to 20 alkoxy moieties and a C₃ to C₁₂ alkyl will increase the volume of foam, but a maximum will be reached at which amounts above this level will then reduce the volume of foam. For reducing stringiness, the addition of an alkoxylated alkyl ether having 5 to 20 alkoxy moieties and a C₃ to C₁₂ alkyl can reduce the time to break to 1.5 seconds or less.

The described cleansing composition includes a cleansing effective amount of one or more anionic surfactants, amphoteric surfactants, nonionic surfactants, cationic surfactants, and combinations thereof. According to the present invention, the surfactant comprises an anionic surfactant and an amphoteric surfactant.

A variety of anionic surfactants can be utilized in the composition including, for example, long chain alkyl (C₆-C₂₂) materials such as alkyl sulfates, alkyl sulfonates, alkyl phosphates, alkyl ether sulfates, alkyl alpha olefin sulfonates, alkyl taurates, alkyl isethionates (SCI), alkyl glyceryl ether sulfonates (AGES), sulfosuccinates, fatty acid soap and the like. These anionic surfactants can be alkoxylated, for example, ethoxylated, although alkoxylation is not required. Examples of classes of anionic surfactants include, but are not limited to, alkyl and alkyl ether sulfates, such as those that may have the respective formula ROSO₃M and RO(C₂H₄O)ₓSO₃M, wherein R is alkyl or alkenyl of from 8 to 24 carbon atoms, x is 1 to 10, and M is a water-soluble cation such as ammonium, sodium, potassium and triethanolamine. The alkyl ether sulfates may be made as condensation products of ethylene oxide and monohydric alcohols having from 8 to 24 carbon atoms. In one embodiment, R has from 10 to 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with 1 to 10, or 3 to 5, or with 3, molar proportions of ethylene oxide and the resulting mixture of molecular species having, for example, an average of 3 moles of ethylene oxide per mole of alcohol, is sulfated and neutralized. Useful anionic surfactants include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, sodium and potassium salts of sodium pareth sulfate, sodium and potassium salts of sodium pareth ether sulfate and combinations thereof.

The fatty acid soap can be any of the neutralized fatty acids, such as those having 8 to 22 carbon atoms. Typical fatty acids used for soaps include, myristic acid, lauric acid, palmitic acid, stearic acids, and other fatty acids. Sources of fatty acids include coconut oil, palm oil, palm kernel oil, tallow, avocado, canola, corn, cottonseed, olive, hi-oleic sunflower, mid-oleic sunflower, sunflower, palm stearin, palm kernel olein, safflower, and babassu oils. The fatty acids can be neutralized with any base to form a soap. Typical bases include, but are not limited to, sodium hydroxide, potassium hydroxide, and triethanolamine.

Amphoteric/zwitterionic surfactants may also be included in the composition. These surfactants are typically characterized by a combination of high surfactant activity, lather forming and mildness. Amphoteric surfactants include, but are not limited to, derivatives of aliphatic secondary and tertiary amines in which the aliphatic radical can be straight chain or branched and wherein one of the aliphatic substituents contains 8 to 18 carbon atoms and one contains an anionic water solubilizing group, *e.g*., carboxy, sulfonate, sulfate, phosphate, or phosphonate. Examples of such compounds include sodium 3-dodecyaminopropionate, sodium 3-dodecylaminopropane sulfonate, N-alkyl taurines and N-higher alkyl aspartic acids. Other equivalent amphoteric surfactants may be used. Examples of amphoteric surfactants include, but are not limited to, a range of betaines including, for example, high alkyl betaines, such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, sulfobetaines such as coco dimethyl sulfopropyl betaine, stearyl dimethyl sulfopropyl betaine, amido betaines, amidosulfobetaines and the like. Betaines having a long chain alkyl group, particularly coco, may be particularly useful as are those that include an amido groups such as the cocamidopropyl and cocoamidoethyl betaines.

Examples of nonionic surfactants include, but are not limited to, sorbitan esters, long chain alkyl glucosides having C₈-C₂₂ alkyl groups; coconut fatty acid monoethanolamides such as cocamide MEA; coconut fatty acid diethanolamides, fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (for example the PLURONIC™ block copolymers commercially available from BASF); fatty acid alkylolamides, (fatty acid amide polyethylene glycols); N-alkyl-, N-alkoxypolyhydroxy fatty acid amides; sucrose esters; sorbitol esters; polyglycol ethers; and combinations thereof.

Cationic surfactants can also be included in the composition. Examples of cationic surfactants include, but are not limited to any quaternium or polyquaternium compound.

Surfactants can be included in any desired amount. In one embodiment, surfactants are present in the composition in an amount of at least 1% by weight, optionally at least 2, at least 3, at least 4, or at least 5% by weight of the composition. The upper amount can be any typical amount for each type of cleansing composition. A cleansing effective amount is any amount that is typically used for body washes/shower gels or liquid hand soaps.

When made into an aqueous liquid cleanser, such as a body wash/shower gel or liquid hand soap, surfactants are typically included in amounts up to 40% by weight of the composition. In certain embodiments, the amount is at least 1, at least 2, at least 3, at least 4, at least 5, at least 6, at least 7, at least 8, at least 9, or 10 up to 40% by weight of the composition. In certain embodiments, the amount is at least 3% or at least 5% up to 40% by weight of the composition.

According to the present invention, the cleansing composition contains an anionic surfactant and an amphoteric surfactant, such as sodium laureth sulfate and cocamidopropyl betaine, and optionally a nonionic surfactant, such as cocamide monoethanolamides. Optionally, the cleansing composition can further include a fatty acid soap.

Other ingredients that can be added to body washes/shower gels or liquid hand soaps can be included in the cleansing composition. In certain embodiments, body washes/shower gels or liquid hand soaps can contain water in an amount of at least 20% by weight of the composition. In other embodiments, the amount of water is at least 30, at least 40, at least 50, at least 60, at least 70, at least 80, or at least 90% by weight of the composition.

Any of the cleansing compositions can be used in a method to cleanse skin. After cleansing, the skin can be rinsed with water.

### EXAMPLES

The following formula is used for a body wash composition in the examples.

| Material | Weight % |
|---|---|
| Sodium laureth sulfate | 7.7 |
| Cocamidopropyl betaine | 3.8 |
| Glycerin | 6 |
| Acrylate copolymer | 2.2 |
| Sodium Chloride | 1 |
| Castor oil maleate | 0.55 |
| Water and minors (color, fragrance, preservatives) | Q.S. |

Viscosity is measured using a Brookfield DV-II viscometer with #4 spindle at 20 rpm at room temperature (23-25°C).

### Example 1

Stringiness reduction of a body wash with PPG-14 butyl ether is measured as the time it takes for a stream of body wash to break once the flow is stopped. Stringiness is measured by videotaping the process of squeezing body wash from a bottle upside down by applying consistent force, followed by analyzing the stringiness time using movie maker software. Ten replicates are obtained for each sample, and the average is called the stringiness time, i.e. the time it takes from the product being squeezed out from the bottle until the liquid filament gets broken apart. The times to break for a body wash and various body washes with different levels of PPG-14 butyl ether are shown in the table below. It can be seen that the PPG-14 butyl ether can reduce the stringiness while keeping the viscosity within 10% of the original value.

| Sample | Time to break (s) | Viscosity (mPas) |
|---|---|---|
| Body wash * | 1.7 | 15980 |
| Body wash with 0.025 wt. % PPG-14 butyl ether* | 1.6 | 15840 |
| Body wash with 0.05 wt. % PPG-14 butyl ether* | 1.5 | 15100 |
| Body wash with 0.1 wt. % PPG-14 butyl ether | 1.2 | 14840 |
| Body wash with 0.3 wt. % PPG-14 butyl ether | 1.3 | 15380 |

| | | |
|---|---|---|
| * Comparative | | |

### Example 2

Foam volume and fragrance release are evaluated by observation by five experienced panelists to compare a body wash without PPG-14 butyl ether to a composition with either 0.1% by weight or 0.3% by weight PPG-14 butyl ether. The following procedure is used.
3. Panelist twists the pouf with the test product 5 times into a plastic bowl, add 5ml of water to the pouf.
4. Panelist again twists the pouf 10 times into the plastic bowl to collect any foam.
5. Panelist evaluates the test products for fragrance intensity.
6. Repeat steps 1 to 5 for the second test product

For the 0.1% by weight PPG-14 butyl ether formula, 3 out of the 5 panelists observed a higher volume of foam from the PPG-14 butyl ether formula as compared to the control body wash without PPG-14 butyl ether, while 2 panelists rated the foam volume as parity. For fragrance, 4 out of 5 panelists rated the fragrance intensity from the PPG-14 butyl ether composition as higher compared to the control body wash, while one panelist rated the fragrance intensity as parity.

For the 0.3% by weight PPG-14 butyl ether formula, 4 out of the 5 panelists observed a higher volume of foam from the PPG-14 butyl ether formula as compared to the control body wash without PPG-14 butyl ether, while one panelist rated the foam volume as parity. For fragrance, 4 out of 5 panelists rated the fragrance intensity from the PPG-14 butyl ether composition as higher compared to the control body wash, while one panelist rated the fragrance intensity as parity.

The comparative results above show that higher foam volume and higher fragrance intensity can be observed in compositions containing PPG-14 butyl ether.

A comparative test is conducted between PPG-14 butyl ether (of the invention) and PPG-3 myristyl ether (outside the scope of the invention). Example 1 is repeated using 0.3 weight % PPG-14 butyl ether and 0.3 weight % PPG-3 myristyl ether. The results are in the table below. The PPG-3 myristyl ether has similar results to the body wash without any additional material. The PPG-3 myristyl ether is not able to reduce the stringiness as does the PPG-14 butyl ether.

| Sample | Time to break (s) |
|---|---|
| Body wash | 1.7 |
| Body wash with 0.3 wt. % PPG-3 myristyl ether | 1.8 |
| Body wash with 0.3 wt. % PPG-14 butyl ether | 1.2 |

### Example 3

In this example, the increased ability of PPG-14 butyl ether to solubilize a fragrance oil composition is evaluated by measuring the percent transmission of light through a sample (turbidity). The higher the level of transmittance, the better that the fragrance oil is solubilized in (turbidity). The higher the level of transmittance, the better that the fragrance oil is solubilized in the micellar composition without phase separation. The body wash composition above is used, and fragrance is included in various amounts listed in the table below. Percent transmission on a Turbiscan instrument is measured for the body wash without any propoxylated alkyl ether, a body wash with 0.3 weight % PPG-14 butyl ether, and a body wash with 0.3 weight % PPG-3 myristyl ether, in the absence and presence of different fragrance levels. In the table below, it can be seen that higher levels of fragrance oils can be included in a composition with PPG-14 butyl ether and maintain transmittance, which correlates to higher solubilization.

| Weight % Fragrance | % Transmittance | | |
|---|---|---|---|
| | Control Body Wash | Body Wash with 0.3 wt.% PPG-14 butyl ether | Body Wash with 0.3% PPG-3 myristyl ether |
| 0 | 87.245 | 89.935 | 90.475 |
| 1 | 88.255 | 88.255 | 89.95 |
| 2 | 86.325 | Not measured | Not measured |
| 2.5 | 86.06 | Not measured | 87.605 |
| 2.8 | 84.24 | Not measured | 85.89 |
| 3 | 0.0647 | 87.73 | 0.04505 |
| 3.5 | 0.0855 | Not measured | 0.02135 |
| 4 | 0.0956 | 86.345 | Not measured |
| 6 | 0.0018285 | 50.075 | 0.003789 |

### Example 4

Example 3 is repeated but isopropyl myristate is used as the oil instead of fragrance oils. The results are in the table below. Again it can be seen that PPG-14 butyl ether allows for higher solubilization as measured by transmittance.

| Weight % Isopropyl Myristate | % Transmittance | |
|---|---|---|
| | Control Body Wash | Body Wash with 0.3 wt.% PPG-14 butyl ether |
| 0 | 89.315 | 89.985 |
| 1 | 88.905 | 89.155 |
| 2.5 | 59.225 | 86.655 |
| 3 | 32.315 | 70.33 |
| 3.2 | 0.03821 | 49.325 |
| 3.5 | 0.01634 | 26.56 |

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

## Claims

1. A liquid cleansing composition comprising a cleansing effective amount of surfactant and an alkoxylated alkyl ether having 5 to 20 alkoxy moieties and a C₃ to C₁₂ alkyl, wherein the alkoxylated alkyl ether is present in the composition in an amount of 0.1 to 0.5% by weight of the composition, and wherein the surfactant comprises an anionic surfactant and an amphoteric surfactant.

2. The liquid cleansing composition of any preceding claim, wherein the alkoxy is PEG or PPG.

3. The liquid cleansing composition of any preceding claim, wherein the alkoxy is PPG.

4. The liquid cleansing composition of any preceding claim, wherein the alkyl is C₃ to C₆, optionally butyl.

5. The liquid cleansing composition of any preceding claim, wherein the alkoxylated alkyl ether has 12 to 20 alkoxy moieties, optionally 12 to 16 alkoxy moieties.

6. The liquid cleansing composition of any preceding claim, wherein the alkoxylated alkyl ether is PPG-14 butyl ether.

7. The liquid cleansing composition of any preceding claim, wherein the surfactant comprises sodium laureth sulfate and cocamidopropyl betaine.

8. The liquid cleansing composition of any of any preceding claim, wherein an amount of surfactant is at least 3% by weight of the composition, optionally, at least 5%, or at least 10% by weight of the composition.

9. The liquid cleansing composition of any preceding claim, wherein the amount of alkoxylated alkyl ether is 0.1 to 0.3% by weight of the composition.

10. The liquid cleansing composition of any preceding claim, wherein water is present in an amount of at least 20% by weight of the composition, optionally at least 30, at least 40, at least 50, at least 60, at least 70, at least 80, or at least 90% by weight of the composition.

11. A method of cleansing skin comprising cleansing the skin with the liquid cleansing composition of any preceding claim, and optionally rinsing the skin with water.

12. A method for reducing stringiness of an aqueous, liquid cleansing composition, increasing fragrance bloom from an aqueous, liquid cleansing composition, and/or increasing foam volume of an aqueous, liquid cleansing composition, comprising adding an alkoxylated alkyl ether having 5 to 20 alkoxy moieties and a C₃ to C₁₂ alkyl, to the aqueous, liquid cleansing composition in an amount of from 0.1 to 0.5% by weight of the composition.

13. The method of claim 12, wherein the alkoxylated alkyl ether is PPG-14 butyl ether.

14. Use of an alkoxylated alkyl ether having 5 to 20 alkoxy moieties and a C₃ to C₁₂ alkyl in an aqueous, liquid cleansing composition for at least one of: reducing stringiness of the cleansing composition, increase fragrance bloom from the cleansing composition, and increasing foam volume of the cleansing composition, wherein the alkoxylated alkyl ether is present in the composition in an amount of 0.1 to 0.5% by weight of the composition.

## Patentansprüche

1. Flüssige Reinigungszusammensetzung, die eine reinigungswirksame Menge an Tensid und einem alkoxylierten Alkylether mit 5 bis 20 Alkoxyeinheiten und einem C₃- bis C₁₂-Alkyl umfasst, wobei der alkoxylierte Alkylether in der Zusammensetzung in einer Menge von 0,1 bis 0,5 Gew.-% der Zusammensetzung vorliegt und wobei das Tensid ein anionisches Tensid und ein amphoteres Tensid umfasst.

2. Flüssige Reinigungszusammensetzung nach einem vorhergehenden Anspruch, wobei das Alkoxy PEG oder PPG ist.

3. Flüssige Reinigungszusammensetzung nach einem vorhergehenden Anspruch, wobei das Alkoxy PPG ist.

4. Flüssige Reinigungszusammensetzung nach einem vorhergehenden Anspruch, wobei das Alkyl C₃- bis C₆-Alkyl, gegebenenfalls Butyl ist.

5. Flüssige Reinigungszusammensetzung nach einem vorhergehenden Anspruch, wobei der alkoxylierte Alkylether 12 bis 20 Alkoxyeinheiten, gegebenenfalls 12 bis 16 Alkoxyeinheiten aufweist.

6. Flüssige Reinigungszusammensetzung nach einem vorhergehenden Anspruch, wobei der alkoxylierte Alkylether PPG-14-Butylether ist.

7. Flüssige Reinigungszusammensetzung nach einem vorhergehenden Anspruch, wobei das Tensid Natriumlaurethsulfat und Cocamidopropylbetain umfasst.

8. Flüssige Reinigungszusammensetzung nach einem vorhergehenden Anspruch, wobei eine Menge an Tensid mindestens 3 Gew.-% der Zusammensetzung, gegebenenfalls mindestens 5 Gew.-% oder mindestens 10 Gew.-% der Zusammensetzung beträgt.

9. Flüssige Reinigungszusammensetzung nach einem vorhergehenden Anspruch, wobei die Menge an alkoxyliertem Alkylether 0,1 bis 0,3 Gew.-% der Zusammensetzung beträgt.

10. Flüssige Reinigungszusammensetzung nach einem vorhergehenden Anspruch, wobei Wasser in einer Menge von mindestens 20 Gew.-% der Zusammensetzung, gegebenenfalls mindestens 30, mindestens 40, mindestens 50, mindestens 60, mindestens 70, mindestens 80 oder mindestens 90 Gew.-% der Zusammensetzung vorliegt.

11. Verfahren zur Reinigung von Haut, das das Reinigen der Haut mit der flüssigen Reinigungszusammensetzung nach einem vorhergehenden Anspruch und gegebenenfalls das Abspülen der Haut mit Wasser umfasst.

12. Verfahren zur Verringerung der Zähflüssigkeit einer wässrigen flüssigen Reinigungszusammensetzung, Steigerung der Duftstoffentfaltung von einer wässrigen flüssigen Reinigungszusammensetzung und/oder Erhöhung des Schaumvolumens einer wässrigen flüssigen Reinigungszusammensetzung, wobei das Verfahren das Zugeben eines alkoxylierten Alkylethers mit 5 bis 20 Alkoxyeinheiten und einem C₃- bis C₁₂-Alkyl zu der wässrigen flüssigen Reinigungszusammensetzung in einer Menge von 0,1 bis 0,5 Gew.-% der Zusammensetzung umfasst.

13. Verfahren nach Anspruch 12, wobei der alkoxylierte Alkylether PPG-14-Butylether ist.

14. Verwendung eines alkoxylierten Alkylethers mit 5 bis 20 Alkoxyeinheiten und einem C₃- bis C₁₂-Alkyl in einer wässrigen flüssigen Reinigungszusammensetzung zu mindestens einem der Folgenden: Verringerung der Zähflüssigkeit der Reinigungszusammensetzung, Steigerung der Duftstoffentfaltung von der Reinigungszusammensetzung und Erhöhung des Schaumvolumens der Reinigungszusammensetzung, wobei der alkoxylierte Alkylether in der Zusammensetzung in einer Menge von 0,1 bis 0,5 Gew.-% der Zusammensetzung vorliegt.

## Revendications

1. Une composition nettoyante liquide comprenant une quantité nettoyante efficace de tensioactif et d'un éther d'alkyle alcoxylé ayant 5 à 20 fractions alcoxy et un groupe alkyle en C₃ à C₁₂, dans laquelle l'éther d'alkyle alcoxylé est présent dans la composition dans une quantité comprise entre 0,1 et 0,5 % en poids de la composition, et dans laquelle le tensioactif comprend un tensioactif anionique et un tensioactif amphotérique.

2. La composition nettoyante liquide selon l'une quelconque des revendications précédentes, dans laquelle l'alcoxy est du PEG ou du PPG.

3. La composition nettoyante liquide selon l'une quelconque des revendications précédentes, dans laquelle l'alcoxy est du PPG.

4. La composition nettoyante liquide selon l'une quelconque des revendications précédentes, dans laquelle l'alkyle est de C₃ à C₆, en option du butyle.

5. La composition nettoyante liquide selon l'une quelconque des revendications précédentes, dans laquelle l'éther d'alkyle alcoxylé a entre 12 et 20 fractions alcoxy, en option entre 12 et 16 fractions alcoxy.

6. La composition nettoyante liquide selon l'une quelconque des revendications précédentes, dans laquelle l'éther d'alkyle alcoxylé est de l'éther butylique de PPG-14.

7. La composition nettoyante liquide selon l'une quelconque des revendications précédentes, dans laquelle le tensioactif comprend du sodium laureth sulfate et de la bétaïne de cocamidopropyle.

8. La composition nettoyante liquide selon l'une quelconque des revendications précédentes, dans laquelle la quantité de tensioactif est au moins de 3 % en poids de la composition, en option au moins de 5 % ou au moins de 10 % en poids de la composition.

9. La composition nettoyante liquide selon l'une quelconque des revendications précédentes, dans laquelle la quantité d'éther d'alkyle alcoxylé est comprise entre 0,1 et 0,3 % en poids de la composition.

10. La composition nettoyante liquide selon l'une quelconque des revendications précédentes, dans laquelle l'eau est présente dans une quantité égale à au moins 20 % en poids de la composition, en option au moins 30, au moins 40, au moins 50, au moins 60, au moins 70, au moins 80, au moins 90 % en poids de la composition.

11. Un procédé de nettoyage de la peau consistant à nettoyer la peau avec la composition nettoyante liquide selon la revendication 1, et en option à rincer la peau avec de l'eau.

12. Un procédé destiné à réduire la consistance fibreuse d'une composition nettoyante liquide aqueuse, à augmenter le dégagement de parfum d'une composition nettoyante liquide aqueuse et/ou à augmenter le volume de mousse d'une composition nettoyante liquide aqueuse, consistant à ajouter un éther d'alkyle alcoxylé ayant 5 à 20 fractions alcoxy et un alkyle en C₃ à C₁₂ à la composition nettoyante liquide aqueuse dans une quantité comprise entre 0,1 et 0,5 % en poids de la composition.

13. Le procédé selon la revendication 12, dans lequel l'éther d'alkyle alcoxylé est de l'éther butylique de PPG-14.

14. Une utilisation d'un éther d'alkyle alcoxylé ayant 5 à 20 fractions alcoxy et d'un alkyle en C₃ à C₁₂ dans une composition nettoyante liquide aqueuse pour un ou plusieurs des effets suivants : réduire la consistance fibreuse de la composition nettoyante, augmenter le dégagement de parfum de la composition nettoyante et augmenter le volume de mousse de la composition nettoyante, dans laquelle l'éther d'alkyle alcoxylé est présent dans la composition dans une quantité comprise entre 0,1 et 0,5 % en poids de la composition.
